(19) Europäisches Patentamt
European Patent Office
Office européen des brevets

(11) EP 4 342 452 B1

(12) EUROPEAN PATENT SPECIFICATION

(45) Date of publication and mention of the grant of the patent:
**08.07.2026 Bulletin 2026/28**

(21) Application number: **22853489.7**

(22) Date of filing: **04.08.2022**

(51) International Patent Classification (IPC):
***A61K 9/00*** (2006.01) ***A61K 47/44*** (2017.01)
***A61K 47/10*** (2017.01) ***A61K 31/352*** (2006.01)
***A61P 27/02*** (2006.01) ***A61P 27/04*** (2006.01)
***A61K 9/08*** (2006.01)

(52) Cooperative Patent Classification (CPC):
**A61P 27/02; A61K 9/0048; A61K 9/08; A61K 31/352; A61K 47/10; A61K 47/44; A61P 27/04**

(86) International application number:
**PCT/KR2022/011562**

(87) International publication number:
**WO 2023/014113 (09.02.2023 Gazette 2023/06)**

# (54) EYE DROP COMPOSITION COMPRISING RECOFLAVONE FOR TREATMENT OF XEROPHTHALMIA

AUGENTROPFENZUSAMMENSETZUNG MIT RECOFFLAVON ZUR BEHANDLUNG VON XEROPHTHALMIE

COMPOSITION DE GOUTTES OCULAIRES COMPRENANT DE LA RÉCOFLAVONE DESTINÉE AU TRAITEMENT DE LA XÉROPHTALMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.08.2021 KR 20210103199**

(43) Date of publication of application:
**27.03.2024 Bulletin 2024/13**

(73) Proprietors:
- **GL Pharmtech Corp.**
**Gyeonggi-do 13449 (KR)**
- **Dong-A ST Co., Ltd.**
**Dongdaemun-gu**
**Seoul 02587 (KR)**

(72) Inventors:
- **KIM, Eun Jung**
**Seongnam-si Gyeonggi-do 13449 (KR)**
- **PARK, Jun Sang**
**Seongnam-si Gyeonggi-do 13449 (KR)**

(74) Representative: **Louis Pöhlau Lohrentz**
**Patentanwälte**
**Postfach 30 55**
**90014 Nürnberg (DE)**

(56) References cited:
**KR-A- 20080 107 542** **KR-A- 20100 014 632**
**KR-A- 20190 073 156** **KR-A- 20200 099 547**
**KR-B1- 102 356 603** **US-A1- 2010 130 448**

- **CUCINA ANNAMARIA ET AL: "Dual 0.02% chlorhexidine digluconate - 0.1% disodium EDTA loaded thermosensitive ocular gel forAcanthamoebakeratitis treatment", INTERNATIONAL JOURNAL OF PHARMACEUTICS, vol. 556, 12 December 2018 (2018-12-12), pages 330 - 337, XP085580111, ISSN: 0378-5173, DOI: 10.1016/J.IJPHARM.2018.12.016**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 342 452 B1

## Description

### [Technical Field]

The present invention relates to an ophthalmic composition containing recoflavone for the treatment of dry eye disease, which is a 3',4',5-trimethoxyflavone derivative compound. Specifically, in order to maximize the improvement of symptoms of dry eye, the present invention relates to an ophthalmic composition containing a certain concentration of the active ingredient and at least one of polyoxyethylene castor oil derivatives or poloxamers which are permeation enhancers, so that the active ingredient, recoflavone, can sufficiently penetrate the cornea *in vivo* to exhibit significant therapeutic effects.

### [Background Art]

Dry eye disease is known as a disease that causes inflammation on an ocular surface and discomfort in eyes due to an increased osmolarity of the tear film and loss of tear homeostasis caused by tear deficiency or excessive tear evaporation. However, there was much controversy regarding the appropriate definition for dry eye until only a few years ago, the Tear Film and Ocular Surface Society (TFOS) defined dry eye disease as follows in 2017: "Dry eye disease is a multifactorial disease of the ocular surface characterized by a loss of homeostasis of the tear film, and accompanied by ocular symptoms, in which tear film instability, hyperosmolarity, inflammation and damage of ocular surface, and neurosensory abnormalities play etiological roles. (TFOS DEWS II introduction. Ocul. Surf. 2017, 15, 269-275)." The diagnosis of dry eye disease is performed by using such methods as questionnaires about symptoms, tear volume, tear film breakup time, tear osmolarity, keratoconjunctival staining and the analysis of lipid layer or meibomian gland, and depending on the degree of dry eye disease, artificial tears, anti-inflammatory agent, tear secretion promoters, autologous serum and eyelid treatment are used for the treatment (J. Korean Med. Assoc. 2018, 61(6), 352-364).

For the treatment of dry eye disease, tear replacement approaches with ocular lubricants such as artificial tears are most commonly used and typical examples thereof are eye drops containing sodium hyaluronate or sodium carboxymethylcellulose. For anti-inflammatory therapy, a cyclosporine formulation is used to reduce the immunoactivity markers (e.g., human leukocyte antigen-antigen D related), apoptosis markers (e.g., Fas) and inflammatory cytokines (e.g., interleukin-6) in keratoconjunctival epithelium to treat inflammation on the ocular surface and improve the symptoms of dry eye. The recently developed diquafosol (diquafosol tetrasodium) formulation is a P2Y2 receptor agonist and exhibits the promotion of secretion of aqueous and mucous components of tear. In Japan, a rebamipide suspension formulation has been proven to be effective in increasing the density of goblet cells in eyes and increasing the mucous and aqueous components of tear, and is used as a therapeutic agent for dry eye disease.

Recoflavone is a 3',4',5-trimethoxyflavone derivative compound, $C_{20}H_{18}O_8$, and has a molecular weight of 386.4 by 2-((2-(3,4-dimethoxyphenyl)-5-methoxy-4-oxo-4H-chromen-7-yl)oxy) acetic acid.

Korea Patent Registrations Nos. 10-0447918, 10-0327621 and 10-0644928 disclosed the applicability of recoflavone to gastrointestinal diseases such as gastritis and gastric ulcer, and inflammatory bowel diseases such as Crohn's disease and colitis by the anti-inflammatory effect and the promotion of gastric mucus secretion. Korea Patent Registration No. 10-0930467 disclosed the conjunctival mucus secretion promoting action of 3',4',5-trimethoxyflavone derivative compounds including recoflavone and the applicability as an agent for treating and preventing dry eye disease.

Korea Patent Registration No. 10-0644928 described that recoflavone can be used in the form of an anhydride as it is, but since its hygroscopicity causes problems in handling and management, it can be improved when it is used as a hydrate. US2010/130448A1 discloses Recoflavone for treatment of dry eye syndrome. Studies were done on mucin-secreting effects in the human mucoepidermoid pulmonary carcinoma cell line, and on therapeutic effects in dry eye syndrome-induced rabbits.

Recoflavone is practically insoluble in water. It has a property with low solubility at low pH. As the pH increases, it is ionized and its solubility increases. However, even when it was dissolved, recoflavone exhibits poor cell permeability due to the high degree of ionization and it could have a problem in the absorption into the body. In fact, when a 3% eye drop was administered to rabbit eyes and the drug distribution in each tissue of eye with time was evaluated, the results showed that recoflavone was rarely distributed in the tissues located inside the eye and mainly distributed in the surrounding tissues outside of the eye, such as the cornea, conjunctiva, and sclera. In particular, the results showed that the drug concentration in the cornea was high immediately after the administration, but it was rapidly decreased over time and the drug was mainly distributed in the conjunctiva and sclera (data not shown).

Therefore, it needs that an eye drop formulation of recoflavone can penetrate the keratoconjunctiva *in vivo* in a certain amount or more so that it can exhibit significant therapeutic effects on the overall symptoms of dry eye disease.

[Prior Arts]

[Patent Document]

(Patent Document 1) Korea Patent Registration No. 10-0327621
(Patent Document 2) Korea Patent Registration No. 10-0447918
(Patent Document 3) Korea Patent Registration No. 10-0644928
(Patent Document 4) Korea Patent Registration No. 10-0930467

[Non-patent Document]

(Non-patent Document 1) TFOS DEWS II introduction. Ocul. Surf. 2017, 15, 269-275
(Non-patent Document 2) J. Korean Med. Assoc. 2018, 61(6), 352-364
(Non-patent Document 3) Invest. Ophthalmol. Vis. Sci. 2014, 55(10), 6569~6574

**[Disclosure of Invention]**

**[Technical Problem]**

The present invention relates to an ophthalmic composition containing recoflavone for the treatment of dry eye disease, and provides an ophthalmic composition of which active ingredients can penetrate the keratoconjunctiva *in vivo* in a certain amount or more so that recoflavone can exhibit significant therapeutic effects on the overall symptoms of dry eye disease.

Korea Patent Registration No. 10-0930467 disclosed that recoflavone showed some evidences for the treatment of dry eye disease, such as the promotion of mucus secretion, at a low drug concentration and had the possibility of development as a therapeutic agent of dry eye disease.

Normally, when a scar occurs in the keratoconjunctiva of an eye, the expression of major mucin proteins, such as MUC1, MUC4, MUC16, and MUC5AC, is increased. However, the patients with dry eye disease complain some discomfort because the mucus secretion is not good enough even though it is easy to get scars in their cornea. The pharmacological mucin secretion by recoflavone in human keratoconjunctival epithelial cells was studied and it confirmed that the thickness of the mucin layer was significantly increased when 100 μM solution of the active ingredient was applied. However, real-time Polymerase Chain Reaction (PCR) confirmed that the mucin proteins related with the symptoms of dry eye, which were MUC1, MUC4, MUC16 and MUC5AC, showed a tendency to increase significantly for four hours and then decreased rapidly. The Western blot analysis also showed that MUC5AC only had a tendency to increase and the others did not (Investigative Ophthalmology and Visual Science, 2014, 55(10), 6565-6574). These results suggested that the pharmacological effects of recoflavone could improve only a part of the symptoms of dry eye and the development potential to an efficient therapeutic agent could be restricted when it was considered that dry eye disease has various symptoms caused by various factors.

In the process of developing a formulation containing recoflavone as a therapeutic agent of dry eye disease, it was confirmed that it was difficult to effectively improve the overall symptoms of dry eye in *in vivo* studies with a low drug concentration in a simply dissolved state. It was found through the present invention that the overall symptoms of dry eye could be effectively improved just when recoflavone could permeate into tissues and cells *in vivo* in a certain amount or more. Then it could be developed as a practical therapeutic agent of dry eye disease. Therefore, in the present invention, it is necessary to develop a recoflavone-containing formulation that can efficiently penetrate the cornea to improve the overall symptoms of dry eye significantly in tissues and cells of the eye.

**[Solution to Problem]**

The present invention provides an ophthalmic composition containing recoflavone, as defined by the claims, which efficiently penetrates into the keratoconjunctiva to exhibit significant improvement of the symptoms of dry eye in tissues and cells of the eye. Specifically, the present invention provides an ophthalmic composition wherein:

a) the effective concentration of recoflavone in the eye drop is 5% (w/v) or higher;
b) the ophthalmic composition comprises at least one permeation enhancer, as defined by the claims; and
c) the ophthalmic composition may optionally further comprise viscosity agents, tonicity agents, buffering agents, pH-adjusting agents and preservatives.

## [Advantageous Effects of Invention]

The present invention can effectively improve the symptoms of dry eye disease by increasing the amount of keratoconjunctival permeation *in vivo* of a recoflavone-containing ophthalmic composition for the treatment of dry eye disease. In particular, the present invention provides a practical and efficient ophthalmic composition for the treatment of dry eye disease by improving the overall signs of dry eye disease, such as tear secretion, corneal fluorescence staining score, the detachment of corneal epithelial cells, corneal surface irregularity, conjunctival goblet cell density and mucin cell density.

The effect of the present invention is not limited to the abovementioned effect, and various effect may be included within the range that is obvious to those skilled in the art from the description below.

## [Description of the Drawings]

FIG. 1 shows the cumulative amount of corneal permeation of recoflavone in the cornea of minipig for 6 hours.
FIG. 2 shows a comparison of tear secretion between the ophthalmic compositions of Comparative Example 1 and Example 1 in the experimental dry eye mouse model.
FIG. 3 shows a comparison of the corneal fluorescence staining score between the ophthalmic compositions of Comparative Example 1 and Example 1 in the experimental dry eye mouse model.
FIG. 4 shows a comparison of the corneal surface irregularity between the ophthalmic compositions of Comparative Example 1 and Example 1 in the experimental dry eye mouse model.
FIG. 5 shows a comparison of the number of detached corneal epithelial cells between the ophthalmic compositions of Comparative Example 1 and Example 1 in the experimental dry eye mouse model.
FIG. 6 shows a comparison of goblet cell density between the ophthalmic compositions of Comparative Example 1 and Example 1 in the experimental dry eye mouse model.
FIG. 7 shows a photograph comparing the mucin cell density between the ophthalmic compositions of Comparative Example 1 and Example 1 in the experimental dry eye mouse model.
FIG. 8 shows a graph comparing the mucin cell density between the ophthalmic compositions of Comparative Example 1 and Example 1 in the experimental dry eye mouse model.

## [Best Mode for Carrying out the Invention]

Hereinafter, the present specification will be described in more detail.

The inventors of the present invention figured out in the process of developing a formulation containing recoflavone into a therapeutic agent of dry eye disease that it is difficult to effectively improve the overall symptoms of dry eye in *in vivo* studies with a low drug concentration in a just dissolved state. During the research to develop recoflavone as an effective therapeutic agent of dry eye disease, it was found that when the formulation contains 5% (w/v) or more of recoflavone and polyoxyethylene castor oil derivatives or poloxamers, which are permeation enhancers, alone or in combination in an amount of 0.1 to 5.0 % (w/v), the amount of keratoconjunctival permeation *in vivo* is increased to effectively improve the symptoms of dry eye, thereby completing the present invention.

Hereinafter, the present specification will be described in more detail.

As used here in the present specification, containing 5% (w/v) or more of recoflavone or a pharmaceutically acceptable salt thereof, a hydrate, anhydride or solvate thereof means containing 5% (w/v) or more as recoflavone, referring to the concentration contained as recoflavone for both the content described as an example and the content that is similar to the description. In addition, the concentration of recoflavone also refers to the concentration as recoflavone.

### Ophthalmic composition for treating dry eye disease

The ophthalmic composition of the present invention comprises 5% (w/v) or more of recoflavone and one or more permeation enhancers, as defined by the claims. This ophthalmic composition may optionally further comprise viscosity agents, tonicity agents, buffering agents, pH-adjusting agents and preservatives.

Specifically, the present invention provides an ophthalmic composition for treating dry eye disease, the composition containing 5% (w/v) or more of recoflavone or its pharmaceutically acceptable salt thereof; and 0.1 to 5.0% *(w/v)* of polyoxyethylene castor oil derivative or poloxamer alone or in combination.

Recoflavone as the active ingredient of the present invention may be used in the form of an anhydride or a solvate including hydrates, and may also be used as one of its pharmaceutically acceptable salt.

In the present invention, recoflavone may be prepared directly by a conventionally known manufacturing method,

or may be prepared by commercial purchase.

In the present invention, the term "recoflavone" refers to a 3',4',5-trimethoxyflavone derivative compound having the structure of Chemical formula 1 below.

[Chemical formula 1]

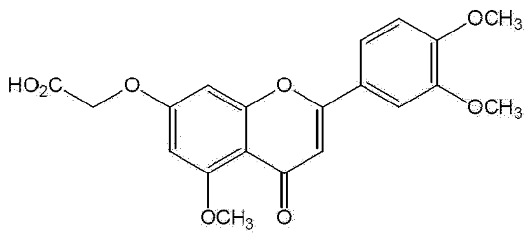

Korean Patent Registration No. 10-0644928 discloses that recoflavone has hygroscopicity in the form of an anhydride as represented by the chemical formula itself, but it is stable without hygroscopicity when it is present in the form of a hydrate, and that it may be present in various solvates. Therefore, it is preferable to use solvates including hydrates in solid dosage forms such as tablet and capsule in the aspect of stability, but in liquid dosage forms such as eye drop, various forms of recoflavone such as anhydrides, solvates including hydrates and its pharmaceutically acceptable salts can be available.

With regard to the concentration of recoflavone, Korean Patent Registration No. 10-0930467 and the prior arts implemented 1 to 3% (w/v) as an active ingredient. However, it was found in the present invention that a certain amount or more of recoflavone must penetrate the tissue *in vivo* in order to improve significantly overall symptoms of dry eye and, in this case, the concentration of recoflavone should be 5% (w/v) or more in a formulation.

Preferably, the concentration of recoflavone is 5% (w/v) or more and 10% (w/v) or less. More than 10% (w/v) of recoflavone is difficult to apply to an eye drop formulation due to osmolarity.

In the present invention, the "permeation enhancer" refers what plays a role of permeating a certain amount or more of recoflavone in the cornea *in vivo* so as to improve significantly overall symptoms of dry eye, when an ophthalmic composition containing recoflavone is administered to the eye, and it may be a kind of surfactant that plays a role of enhancing the cell membrane permeation of recoflavone.

Specifically, the permeation enhancer includes polyoxyethylene castor oil derivatives or poloxamers, and more specifically, the polyoxyethylene castor oil derivative may be polyoxyl 35 castor oil, and the poloxamer may be poloxamer 407.

In the present invention, the polyoxyethylene castor oil derivative or poloxamer may be used alone or in combination, and when included alone or in combination, it may be included in a range of 0.1 to 5.0% (w/v) in the ophthalmic composition.

In the present invention, a viscosity agent that is commonly used for eye drops may be optionally comprised, if necessary. For example, nonionic polymers such as polyvinylpyrrolidone, polyvinyl alcohol, hydroxypropylmethylcellulose, hydroxyethylcellulose, methylcellulose; ionic polymers such as sodium carboxymethylcellulose, polycarbophil, carbomer; sugars such as glucan, chitosan, trehalose; and gums such as xanthan gum can be used, and may be optionally comprised in a range of 0 to 3.0% (w/v) in the ophthalmic composition.

In the present invention, a tonicity agent that is commonly used for isotonicity with tear may be used. For example, nonionic compounds such as mannitol, glycerin, propylene glycol, polyethylene glycol, maltose, sucrose, sorbitol, trehalose and glucose or ionic compounds such as sodium chloride, sodium nitrate and potassium nitrate may be used. These tonicity agents may be used alone or in any combination of two or more thereof to adjust osmotic pressure to be similar to that of tear, and may be included in a range of 0.01 to 1.0% (w/v) in the ophthalmic composition.

In the present invention, in order to adjust and maintain the pH in a range that does not irritate or damage the cornea or conjunctiva, a buffer agent or a pH-adjusting agent that is commonly used in the field of ophthalmic formulations may be used. For example, phosphate, citrate, acetate, hydrogen carbonate, carbonate, gluconate, lactate, propionate, TRIS or borate may be used as a buffer agent, and conventional acids such as hydrochloric acid, lactic acid, acetic acid, phosphoric acid and citric acid or conventional bases such as sodium hydroxide and potassium hydroxide may be used as a pH-adjusting agent alone or in any combination of two or more thereof. In this case, it may be included in a range of 0.01 to 3.0% (w/v) in the ophthalmic composition.

In the present invention, for the purpose of preventing the eye drop from contamination with bacteria or fungi, a preservative that is commonly used in eye drops may be optionally used, if necessary, and it usually depends on whether a multi-use container or a disposable container is used as a package of an ophthalmic formulation. For example, tertiary

ammonium salts such as benzalkonium chloride and benzethonium chloride; parahydroxybenzoic acid esters such as methylparaben and propylparaben; alcohols such as chlorobutanol and benzyl alcohol; chlorhexidine acetate and sodium edetate may be used alone or in any combination of two or more thereof, and may be optionally included in a range of 0 to 0.1 % (w/v) in the ophthalmic composition.

In the present invention, for the ophthalmic composition containing recoflavone for the treatment of dry eye disease, the cumulative permeation amount of recoflavone in the cornea of minipig for 6 hours is 130 $\mu g/cm^2$ or more, or 3 times or more compared to an ophthalmic composition that does not contain a permeation enhancer. In this case, the cumulative permeation amount of recoflavone means the total amount of recoflavone that has permeated the cornea of minipig for 6 hours when tested according to Experimental Example 1.

Because the cornea has a function of protecting the eyes and acts as a barrier to drug absorption, it is necessary to secure an appropriate corneal permeation amount of drug to successfully develop an eye drop.

Recoflavone has the advantage of being able to prepare an eye drop in a fully transparent solution even at a high concentration of 3% (w/v) or 5% (w/v). However, the eye drops containing recoflavone that is just dissolved at a high concentration failed to show a significant improvement of the overall symptoms of dry eye disease in *in vivo* studies, and the results of the corneal permeability test confirmed that recoflavone could not penetrate in a sufficient amount although recoflavone was dissolved at a high concentration. In the present invention, it was confirmed that the corneal permeation amount of recoflavone was significantly increased when a permeation enhancer was included in the recoflavone-containing ophthalmic composition, which induced the improvement of overall symptoms of dry eye disease.

In the present invention, considering the properties of an eye drop administered to eyes directly and the physicochemical properties of recoflavone, the ophthalmic composition for the treatment of dry eye disease may have a pH range of 4.0 to 8.0, and the range may preferably be set to a pH of 5.0 to 7.0.

**Usage and dosage of ophthalmic composition**

For the ophthalmic composition of the present invention, it is advantageous that the active ingredient is sufficiently exposed to the eye so that the drug can be delivered to the inside or outside of the keratoconjunctival cells of the eye in a certain amount or more for significant improvement of the overall symptoms of dry eye. Therefore, the method of administering the ophthalmic composition of the present invention is preferable to instill 1 to 2 drops at a time, 3 times or more a day.

Specifically, 1 drop at a time may be instilled 3 or more, 4 or more, 5 or more, or 6 or more times, and more specifically, 1 drop at a time may be instilled 3 to 6 times a day.

The ophthalmic composition according to the present invention may be administered in combination with other eye drops, and in this case, may be administered at an interval of at least 5 minutes.

Hereinafter, preferred Examples are presented to help the understanding of the present invention. However, the following Examples are provided for easier understanding of the present invention, and the content of the present invention is not limited by the Examples.

**Examples 1 to 4 and Comparative Examples 1 to 2: Preparation of ophthalmic compositions**

An ophthalmic composition was prepared according to the ratio shown in Table 1 below. First, while stirring an appropriate amount of purified water, sodium hydroxide was added to the purified water to dissolve, and then recoflavone monohydrate was added to dissolve. Hydrochloric acid was added to the dissolved solution to adjust to pH 4 to 8, and then a permeation enhancer, a tonicity agent, a buffering agent and a preservative were added to dissolve. A viscosity agent was separately put into in an appropriate amount of purified water and dissolved by heating or by keeping at room temperature depending on the type, and then added to the solution described above and mixed. Additional purified water was applied to make sure the correct volume. After checking if the pH was 4 to 8, the pH was adjusted using a pH-adjusting agent, if necessary. The solution was passed through a filter of 0.22 $\mu m$ or less to prepare a transparent ophthalmic composition.

[Table 1]

| Unit of concentration (%, w/v) | Compar ative Example 1 | Compara tive Example 2 | Example 1 | Example 2 | Example 3 | Example 4 |
|---|---|---|---|---|---|---|
| Recoflavone | 5% | 3% | 5% | 5% | 5% | 5% |
| Polyoxyl 35 castor oil | - | 1% | 1% | - | 5% | 1% |
| Poloxamer 407 | - | - | - | 0.1% | - | 0.2% |
| Polyvinyl alcohol | - | - | - | 0.25% | - | - |

(continued)

| Unit of concentration (%, w/v) | Compar ative Example 1 | Compara tive Example 2 | Example 1 | Example 2 | Example 3 | Example 4 |
|---|---|---|---|---|---|---|
| Hydroxyethyl cellulose | - | - | - | 0.1% | - | - |
| Benzalkonium chloride | - | - | - | - | 0.01% | - |
| Sodium hydroxide | q.s. | q.s. | q.s. | q.s. | q.s. | q.s. |
| Hydrochloric acid | q.s. | q.s. | q.s. | q.s. | q.s. | q.s. |
| Sodium chloride | q.s. | q.s. | q.s. | q.s. | q.s. | q.s. |
| Glycerin | - | - | - | q.s. | - | - |
| Sorbitol | - | - | - | - | q.s. | - |
| Trehalose | - | - | - | - | q.s. | - |
| Purified water | q.s. | q.s. | q.s. | q.s. | q.s. | q.s. |

**Experimental Example 1: Corneal permeability test**

In order to evaluate the degree of permeation of the ophthalmic composition in the keratoconjunctiva, the permeation rate was measured by using the cornea of minipig which is relatively uneasy to permeate and which allows for a corneal permeation test. In general, it is reported that the conjunctiva is 2-3 times easier to permeate compared to the cornea.

The *in vitro* test of corneal permeation for the ophthalmic compositions according to Comparative Examples and Examples was conducted by using a vertical Franz diffusion cell device. The cornea with the surrounding sclera for test was harvested from the eyes of minipigs that weighed 40 to 50 kg. In order to fix the cornea in Franz diffusion cell, the corneal region was only exposed at the center of receptor chamber. Then, the donor chamber was covered and fixed with a joint. Cautions were taken to remove the air from the area where the receptor chamber meets the cornea. 5mL of phosphate solution (PBS buffer) at pH 7.4 and a magnetic stirrer were put into the receptor chamber. 1mL of test solution was put into the donor chamber, which was then closed with a lid and sealed with a parafilm. During the permeability test, the stirrer kept at about 500 rpm, and the Franz diffusion cells kept at 36.5°C. At 0.5, 1, 2, 4 and 6 hours, 200 μL of each sample was taken and the same amount of phosphate solution (PBS buffer) was supplemented. The drug concentration in the collected samples was measured by using an HPLC analysis instrument. The concentration measured in each sample was converted to the permeation amount per time, and the amount of the diluent was corrected to calculate the cumulative permeation amount of recoflavone for 6 hours. The results of *in vitro* permeability test by using the cornea of minipig confirmed that the permeation enhancer which was contained in Examples increased the cumulative permeation amount of recoflavone.

The cumulative permeation amount per unit area in each individual sample was plotted with the average over sampling time, and the linear regression analysis was performed. The slope of equation was calculated as the average permeation rate (flux) of the drug for 6 hours. The relative permeation enhancement ratio was calculated as the ratio of the average permeation rate for 6 hours of each Example compared to Comparative Example 1 (see equation 1).

[Equation 1]

$$\text{Relative permeation enhancement ratio} = \frac{\text{Average permeation rate of each Example}}{\text{Average permeation rate of Comparative Example 1}}$$

Through the above method, the average cumulative permeation amount and the average permeation rate over time per unit area of the cornea, and the relative permeation enhancement ratio of the ophthalmic compositions of

Examples 1 to 3 and Comparative Examples 1 and 2 were calculated and are shown in Table 2.

Referring to Table 2, Examples 1 to 3 confirmed that permeation of the drug was different depending on the presence or absence of a permeation enhancer at the same drug concentration compared to Comparative Example 1 which contains only the active ingredient like a conventional eye drop composition. In addition, Example 1 confirmed that the permeation was enhanced depending on the concentration of recoflavone at the same composition compared to Comparative Example 2.

In addition, it was confirmed that the permeability of Comparative Example 2 which had a lower concentration of recoflavone but had a permeation enhancer was higher than that of Comparative Examples 1.

The results showed that Example 1 had an average permeation rate of 27 $\mu g/cm^2/hr$ for 6 hours and so the relative permeation enhancement ratio increased 3.4 times compared to Comparative Example 1 without a permeation enhancer.

The improvement of the symptoms of dry eye *in vivo* was also comparatively evaluated in Experimental Examples described below, and the results showed that the overall improvement by Example 1 was significantly better than those by Comparative Example 1 without a permeation enhancer.

Therefore, in pursuit of the overall improvement of the symptoms of dry eye *in vivo* rather than simply getting better the laboratory data, it was confirmed that the relative permeation enhancement ratio for 6 hours in the cornea of minipig, compared to Comparative Example 1 without a permeation enhancer, should preferably be 3.0 or higher, or the cumulative permeation amount should preferably be 130 $\mu g/cm^2$ or more.

[Table 2]

| | Comparativ e Example 1 (n=6) | Comparativ e Example 2 (n=6) | Example 1 (n=6) | Example 2 (n=3) | Example 3 (n=3) |
|---|---|---|---|---|---|
| Cumulative permeation amount ($\mu g/cm^2$) | 43.21 | 81.14 | 149.53 | 315.09 | 252.31 |
| Ratio of cumulative permeation amount compared to Comparative Example 1 | 1.00 | 1.88 | 3.46 | 7.29 | 5.84 |
| Average permeation rate ($\mu g/cm^2/hr$) | 7.86 | 14.64 | 27.04 | 56.35 | 45.36 |
| Relative permeation enhancement ratio compared to Comparative Example 1 | 1.00 | 1.86 | 3.44 | 7.17 | 5.77 |

## Experimental Example 2: Tear secretion

It was evaluated the effect of the improved corneal permeation of the ophthalmic composition on the tear secretion in the experimental dry eye mouse model.

NOD.B10.H2$^b$ mice 12 to 14 weeks old (Jackson Laboratory, Bar Harbor, USA) were adapted for 7 days and were injected subcutaneously with 0.5 mg/0.2 mL scopolamine hydrobromide into hindquarters 4 times daily (8 am, 11 am, 2 pm, 5 pm) for 10 days with desiccation stress at below average 40% of ambient humidity. After checking the occurrence of dry eyes, the placebo (the composition without the active ingredient in Example 1), Comparative Example 1 and Example 1 were each instilled into both eyes at a dose of 5 $\mu L$, four times a day for 21 days.

To measure the tear volume, phenol red-impregnated cotton threads were placed in the lateral canthus for 20 seconds. The lengths of the thread in which tear was absorbed were converted into microliters ($\mu L$).

FIG. 2 shows the results of the tear secretion in the experimental dry eye mouse model by the method described above. Example 1 with the increased corneal permeation improved the tear volume, while Comparative Example 1 containing 5% (w/v) recoflavone as same as Example 1 showed little difference of the tear volume from that of the placebo.

## Experimental Example 3: Corneal Fluorescein Staining (CFS)

It was evaluated the effect of the improved corneal permeation of the ophthalmic composition on the corneal fluorescence staining (CFS) in the experimental dry eye mouse model.

On the same condition of Experimental Example 2, the placebo (the composition without the active ingredient in Example 1), Comparative Example 1 and Example 1 were instilled for 10 days and 1 $\mu L$ of 1% fluorescein reagent was applied into the eyes to stain the cornea and the eyes was washed with saline. Images were taken with a digital slit lamp to score and evaluate the degree of epithelial damage (the degree of fluorescence staining). The scores from 1 to 3 points for each five areas of cornea were given and added up.

FIG. 3 shows the results of the corneal fluorescence staining (CFS) as a direct index for the improvement of dry eye disease by the method described above. Example 1 showed significant reduction of the corneal fluorescence staining (CFS) which meant the symptoms of dry eye were significantly improved while Comparative Example 1 exhibited no improvement compared to the placebo.

## Experimental Example 4: Evaluation of corneal surface irregularity

It was evaluated the effect of the improved corneal permeation of the ophthalmic composition on the corneal surface irregularity in the experimental dry eye mouse model.

On the same conditions of experimental Example 2, the placebo (the composition without the active ingredient in Example 1), Comparative Example 1 and Example 1 were instilled for 10 days. The white ring images of the cornea surface were reflected and acquired from the fiber optic ring illuminator of a stereoscopic microscope (SZX7, Olympus). The corneal surface irregularity score was given in the reflected ring: 0 point no distortion; 1 point, distortion in 1 quarter of the ring; 2 points, distortion in 2 quarters; 3 points, distortion in 3 quarters; 4 points, distortion in all 4 quarters; 5 points, severe distortion.

The corneal surface irregularity was evaluated to check the effect on the corneal curing by the method described above (FIG. 4). Example 1 showed the reduction of the corneal surface irregularity by about 30% which indicated that the symptoms of dry eye were significantly improved while Comparative Example 1 did not show any difference from the placebo.

## Experimental Example 5: Detachment of corneal epithelial cell

It was evaluated the effect of the improved corneal permeation of the ophthalmic composition on the detachment of corneal epithelial cell in the experimental dry eye mouse model.

On the same conditions of Experimental Example 2, the placebo (the composition without the active ingredient in Example 1), Comparative Example 1 and Example 1 were instilled for 21 days and the animals were sacrificed.

The surgically excised periocular region and lacrimal gland were fixed in 10% formalin for 3 days and embedded in paraffin. The 5 μm tissue was cut with a microtome and dried for 1 hour on a slide. The tissue was prepared by the Hematoxylin-Eosin (H&E) staining method and the detached epithelial cell was evaluated in the 1 $mm^2$ of corneal region by a virtual microscope (Nanozoomer 2.0RS, Hamamatsu, Japan). In order to compare exactly the degree of improvement of the symptoms of dry eye, the eye tissue at the time right before the drug administration after dry eye was fully induced (initial) and the eye tissue of the normal mouse that was exposed to neither the drug or nor the dry environment stress for 10 days (normal eye) were compared together.

FIG. 5 shows the results of evaluating the detached corneal epithelial cell. While the detached epithelial cell was very little in the normal eye, the detachment was severely increased through the exposure to the dry eye-inducing environment for 10 days (initial). Though Comparative Example 1 exhibited a clear improvement compared to the placebo, Example 1 showed a better result of recovery to the state of the normal eye which indicated that the detached epithelial cell was effectively reduced.

## Experimental Example 6: Conjunctival goblet cell density

It was evaluated the effect of the improved corneal permeation of the ophthalmic composition on the density of goblet cells for the mucus secretion in the experimental dry eye mouse model.

On the same conditions of Experimental Example 5, the paraffin-embedded tissue was sectioned and put on a slide. The staining was performed according to the instructions of the PAS (Periodic-Acid-Schiff) kit and the density of goblet cells was evaluated in the 1 $mm^2$ of conjunctival region by a virtual microscope (Nanozoomer 2.0RS, Hamamatsu, Japan).

FIG. 6 shows the results of evaluating the density of conjunctival goblet cells by the method described above. The conjunctival goblet cells of the initial exhibited a significant reduction through the exposure to the dry eye-inducing environment for 10 days compared to the normal eye. The placebo showed no recovery of the conjunctival goblet cells. Comparative Example 1 exhibited a clear improvement effect compared to the placebo, whereas Example 1 showed further improvement from Comparative Example 1 which exhibited a better result of recovery that was close to the level of the normal eye.

## Experimental Example 7: Distribution of mucins

It was evaluated the effect of the improved corneal permeation of the ophthalmic composition on the distribution of mucins, a major component of mucus, in the experimental dry eye mouse model.

On the same conditions of Experimental Example 5, the paraffin-embedded tissue was sectioned and put on a slide. The staining was performed according to the instructions of the Alcian Blue Stain kit and it was evaluated in the 1 $mm^2$ of corneal and conjunctival regions by a virtual microscope (Nanozoomer 2.0RS, Hamamatsu, Japan).

FIGS. 7 and 8 show the distribution of mucins by the method described above. The mucins of the initial exhibited a significant reduction through the exposure to the dry eye-inducing environment for 10 days compared to the normal eye. The placebo showed no recovery of the mucins which are key components of mucus. Comparative Example 1 exhibited a clear improvement effect compared to the placebo, whereas Example 1 showed further improvement from Comparative Example 1 which exhibited a better result of recovery that was close to the level of the normal eye. These were consistent with the results of the density of goblet cells for the mucin secretion in Experimental Example 6.

## Claims

1. An ophthalmic composition for the treatment of dry eye disease, the composition comprising 5% (w/v) or more of recoflavone or a pharmaceutically acceptable salt thereof; and 0.1 to 5.0% (w/v) of polyoxyethylene castor oil derivative or poloxamer alone or in combination.

2. The ophthalmic composition for the treatment of dry eye disease of claim 1, wherein the polyoxyethylene castor oil derivative is polyoxyl 35 castor oil.

3. The ophthalmic composition for the treatment of dry eye disease of claim 1, wherein the poloxamer is poloxamer 407.

4. The ophthalmic composition for the treatment of dry eye disease of claim 1, wherein the ophthalmic composition for the treatment of dry eye disease comprises 5% *(w/v)* to 10% *(w/v)* of recoflavone or a pharmaceutically acceptable salt thereof.

5. The ophthalmic composition for the treatment of dry eye disease of claim 1, wherein the ophthalmic composition for the treatment of dry eye disease has a pH range from 5.0 to 7.0.

## Patentansprüche

1. Ophthalmologische Zusammensetzung zur Behandlung der trockenes Auge-Erkrankung, wobei die Zusammensetzung 5 % (Gew./Vol.) oder mehr von Recoflavon oder einem pharmazeutisch verträglichem Salz davon und 0,1 bis 5,0 % (Gew./Vol.) von Polyoxyethylen-Rizinusöl-Derivat oder Poloxamer, allein oder in Kombination, umfasst.

2. Ophthalmologische Zusammensetzung zur Behandlung der trockenes Auge-Erkrankung nach Anspruch 1, wobei das Polyoxyethylen-Rizinusöl-Derivat Polyoxyl 35-Rizinusöl ist.

3. Ophthalmologische Zusammensetzung zur Behandlung der trockenes Auge-Erkrankung nach Anspruch 1, wobei das Poloxamer Poloxamer 407 ist.

4. Ophthalmologische Zusammensetzung zur Behandlung der trockenes Auge-Erkrankung nach Anspruch 1, wobei die ophthalmologische Zusammensetzung zur Behandlung der trockenes Auge-Erkrankung 5 % (w/v) bis 10 % (w/v) von Recoflavon oder einem pharmazeutisch verträglichem Salz davon umfasst.

5. Ophthalmologische Zusammensetzung zur Behandlung der trockenes Auge-Erkrankung nach Anspruch 1, wobei die ophthalmologische Zusammensetzung zur Behandlung der trockenes Auge-Erkrankung einen pH-Bereich von 5,0 bis 7,0 aufweist.

## Revendications

1. Une composition ophtalmique pour le traitement de la sécheresse oculaire, la composition comprenant 5 % (p/v) ou plus de récoflavone ou d'un sel pharmaceutiquement acceptable de celle-ci ; et de 0,1 à 5,0 % (p/v) de dérivé d'huile de ricin polyoxyéthylénée ou de poloxamère, seuls ou en combinaison.

2. La composition ophtalmique pour le traitement de la sécheresse oculaire selon la revendication 1, dans laquelle le

dérivé d'huile de ricin polyoxyéthylénée est de l'huile de ricin polyoxyéthylénée 35.

**3.** La composition ophtalmique pour le traitement de la sécheresse oculaire selon la revendication 1, dans laquelle le poloxamère est le poloxamère 407.

**4.** La composition ophtalmique pour le traitement de la sécheresse oculaire selon la revendication 1, dans laquelle la composition ophtalmique pour le traitement de la sécheresse oculaire comprend de 5 % (p/v) à 10 % (p/v) de récoflavone ou d'un sel pharmaceutiquement acceptable de celle-ci.

**5.** La composition ophtalmique pour le traitement de la sécheresse oculaire selon la revendication 1, dans laquelle la composition ophtalmique pour le traitement de la sécheresse oculaire présente une plage de pH de 5,0 à 7,0.

【FIG. 1】

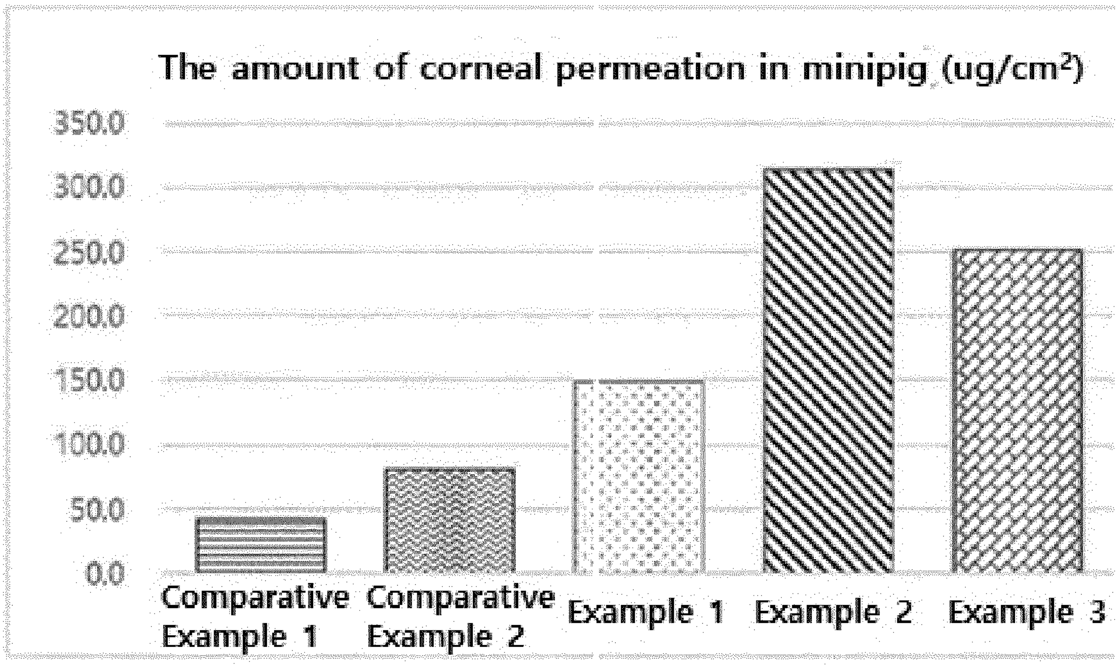
The amount of corneal permeation in minipig (ug/cm2)
350.0
300.0
250.0
200.0
150.0
100.0
50.0
0.0
Comparative Example 1
Comparative Example 2
Example 1
Example 2
Example 3

【FIG. 2】

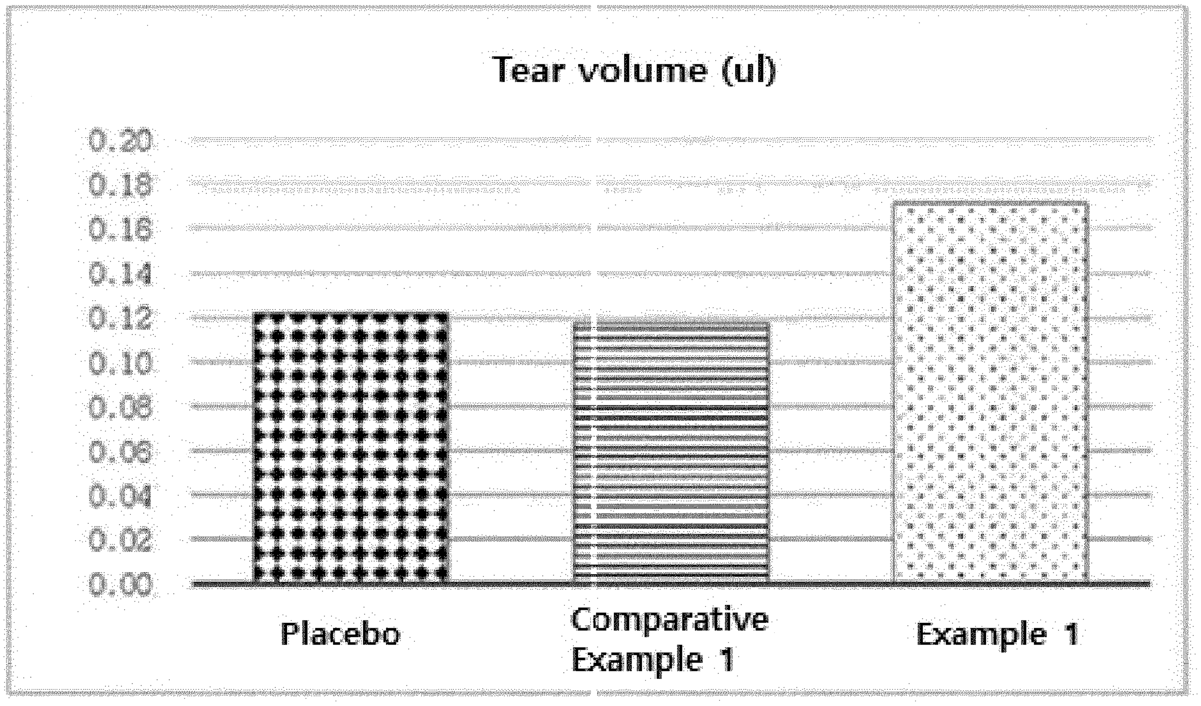
Tear volume (ul)
0.20
0.18
0.16
0.14
0.12
0.10
0.08
0.06
0.04
0.02
0.00
Placebo
Comparative Example 1
Example 1

【FIG. 3】

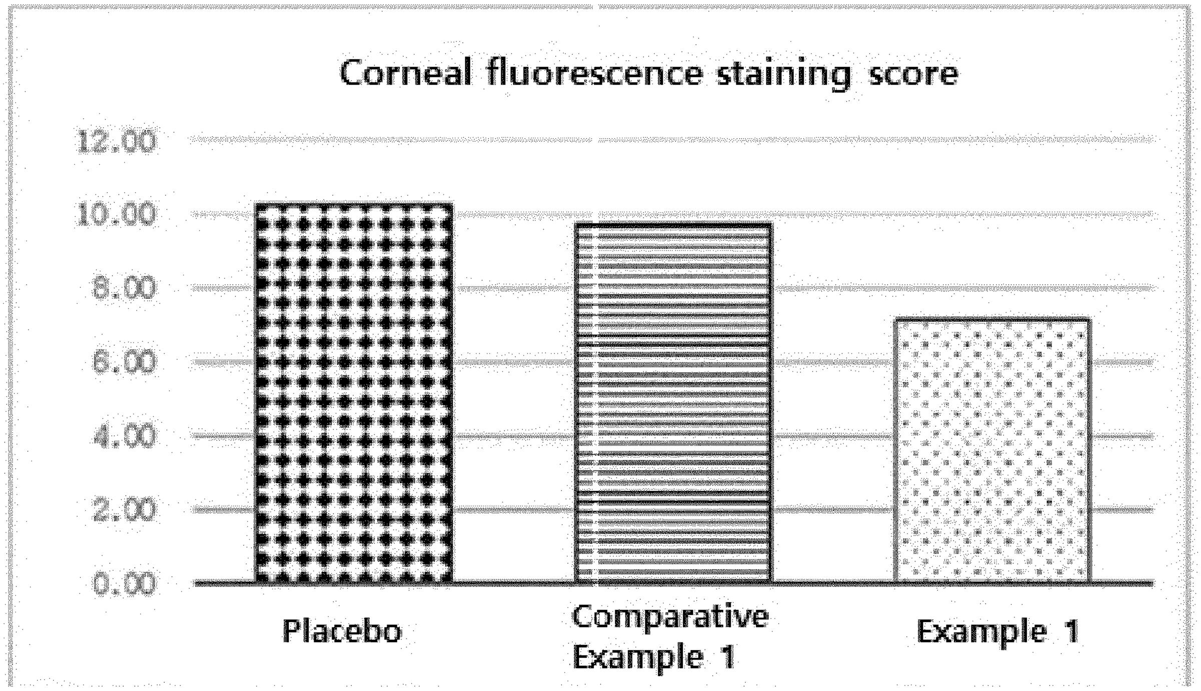
Corneal fluorescence staining score
12.00
10.00
8.00
6.00
4.00
2.00
0.00
Placebo
Comparative Example 1
Example 1

【FIG. 4】

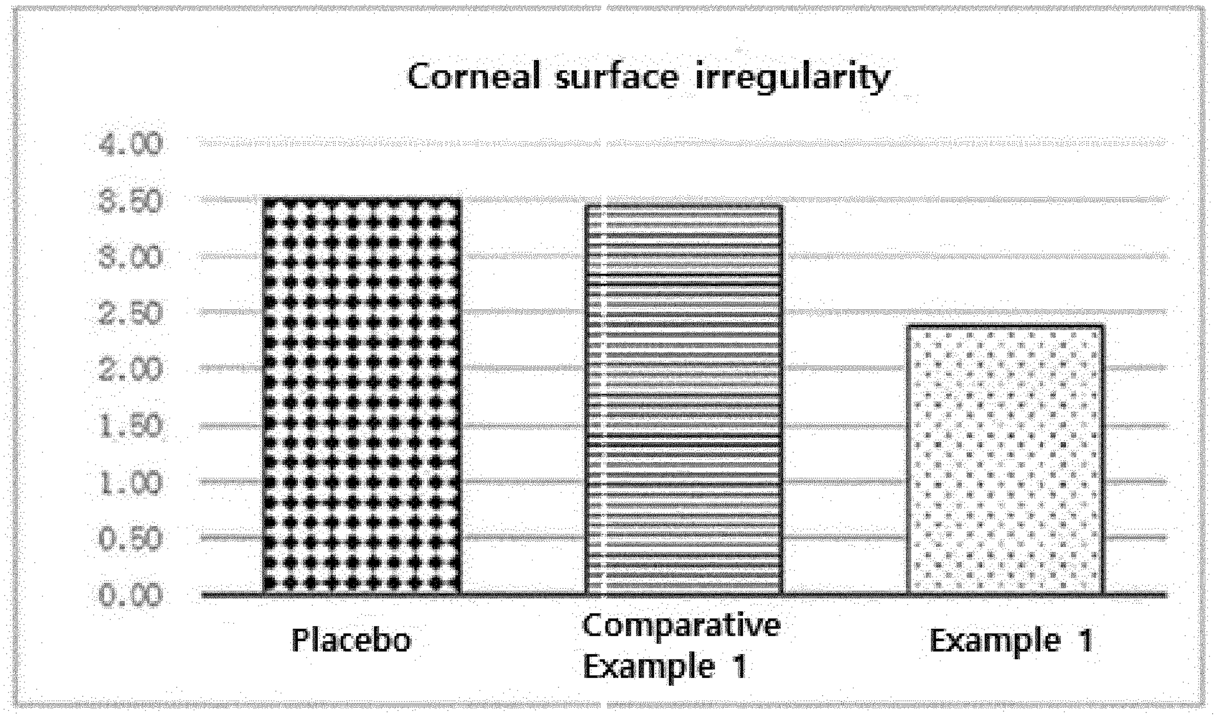
Corneal surface irregularity
4.00
3.50
3.00
2.50
2.00
1.50
1.00
0.50
0.00
Placebo
Comparative Example 1
Example 1

【FIG. 5】

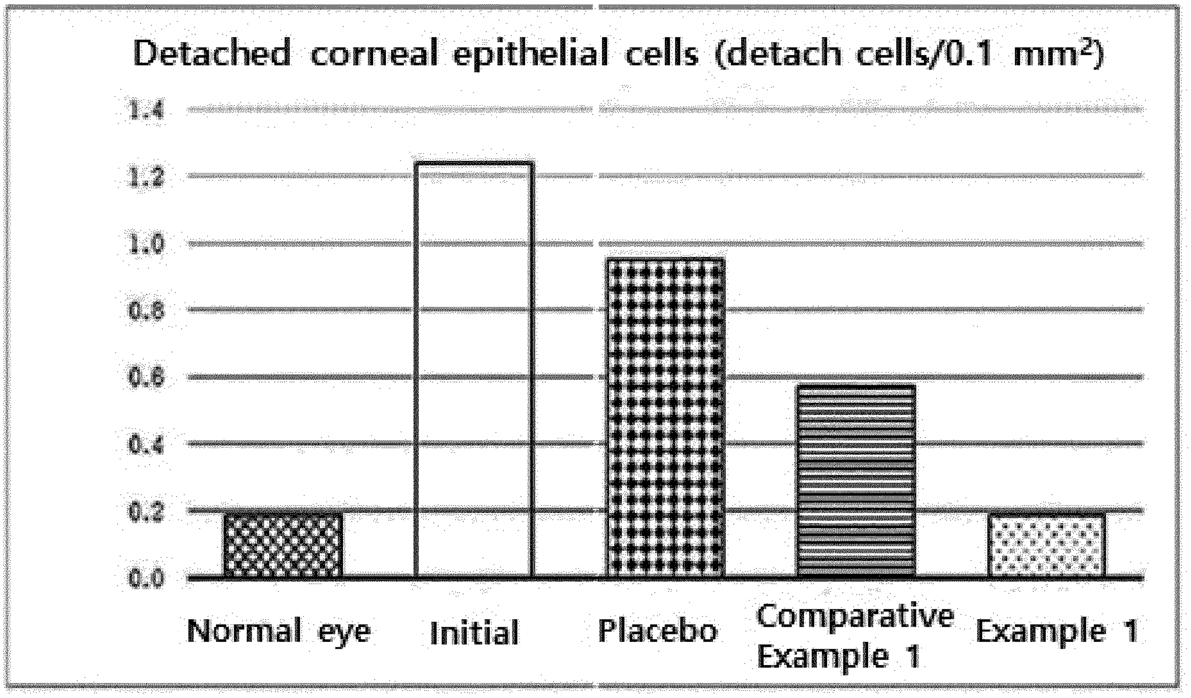
Detached corneal epithelial cells (detach cells/0.1 mm²)
1.4
1.2
1.0
0.8
0.6
0.4
0.2
0.0
Normal eye
Initial
Placebo
Comparative Example 1
Example 1

【FIG. 6】

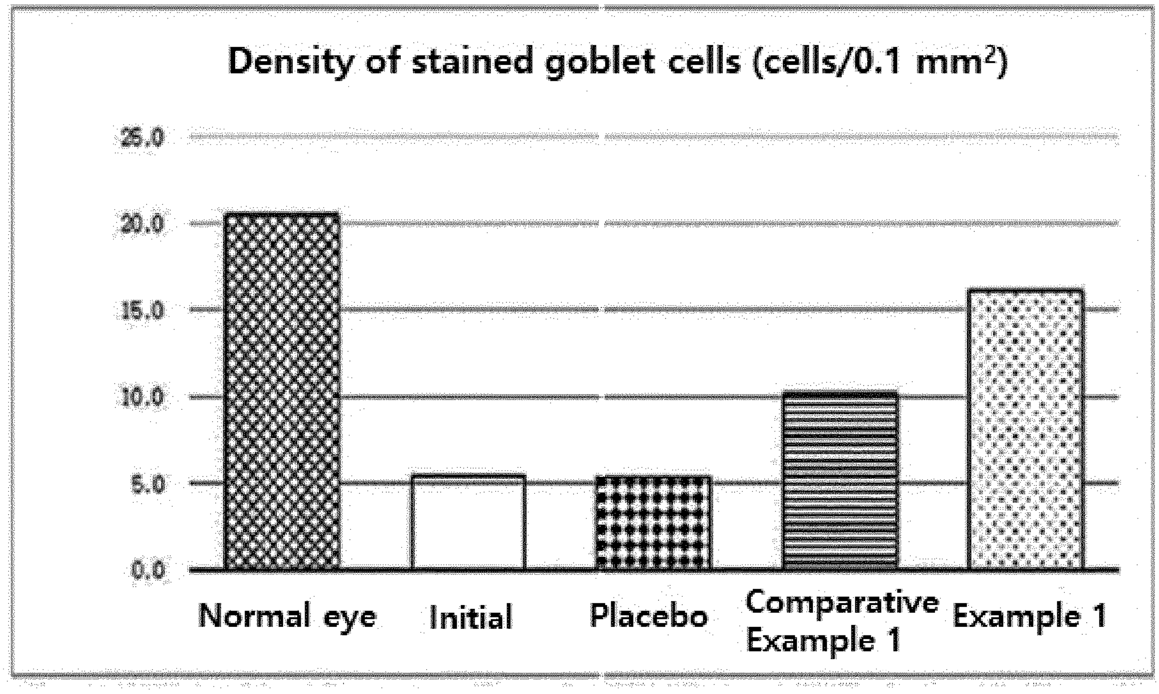
Density of stained goblet cells (cells/0.1 mm2)
25.0
20.0
15.0
10.0
5.0
0.0
Normal eye
Initial
Placebo
Comparative Example 1
Example 1

【FIG. 7】

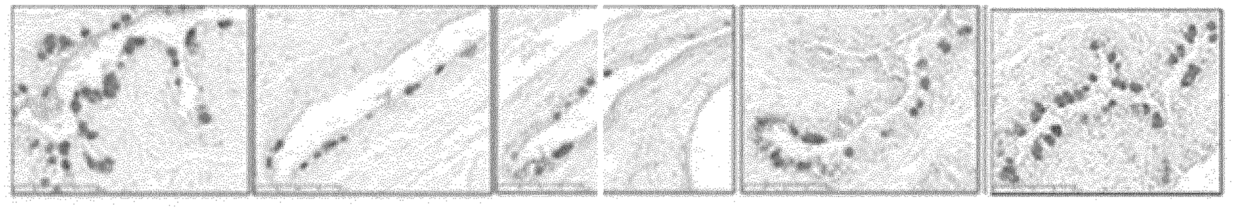

(a) Normal eye (b) Initial (c) Placebo (d) Comparative Example 1 (e) Example 1

【FIG. 8】

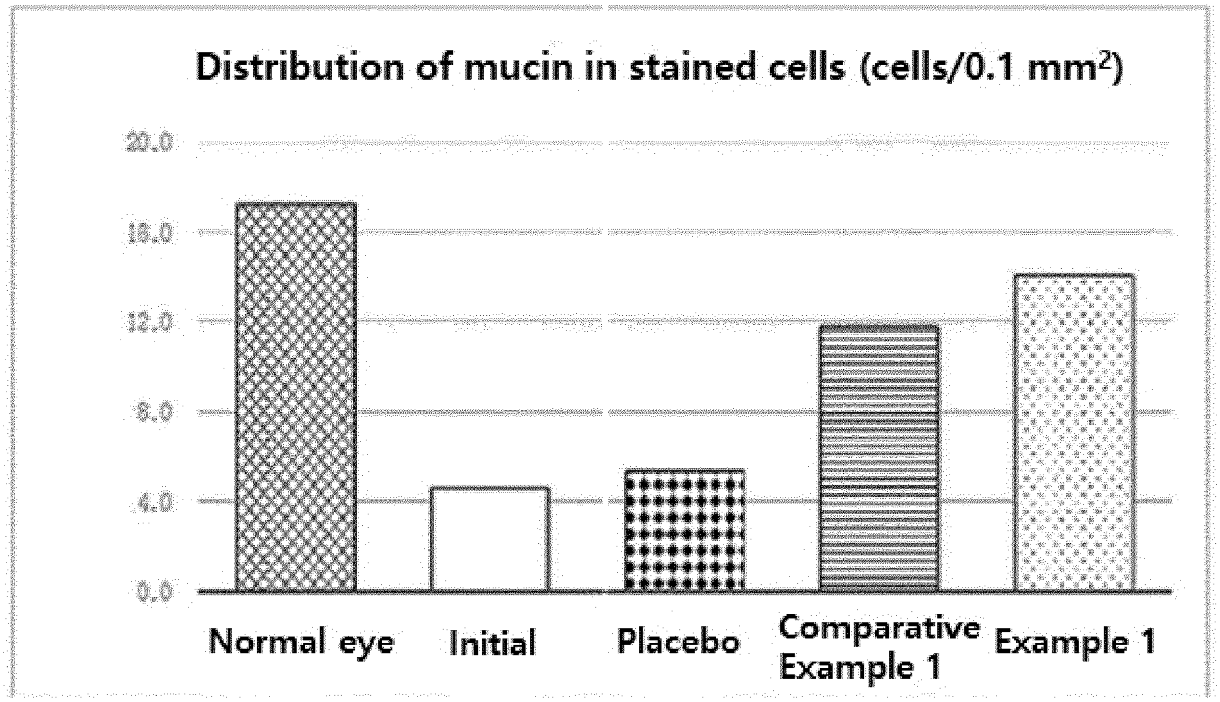
Distribution of mucin in stained cells (cells/0.1 mm2)
20.0
16.0
12.0
8.0
4.0
0.0
Normal eye
Initial
Placebo
Comparative Example 1
Example 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 100447918
- KR 100327621
- KR 100644928
- KR 100930467
- US 2010130448 A1


### Non-patent literature cited in the description


- TFOS DEWS II introduction. *Ocul. Surf.*, 2017, vol. 15, 269-275
- *J. Korean Med. Assoc.*, 2018, vol. 61 (6), 352-364
- *Invest. Ophthalmol. Vis. Sci.*, 2014, vol. 55 (10), 6569-6574
- *Investigative Ophthalmology and Visual Science*, 2014, vol. 55 (10), 6565-6574